(19) Europäisches Patentamt
European Patent Office
Office européen des brevets

(11) Veröffentlichungsnummer: **0 268 553 B1**

# (12) EUROPÄISCHE PATENTSCHRIFT

(45) Veröffentlichungstag der Patentschrift: **11.09.91**

(51) Int. Cl.$^5$: **C07D 235/20**, C07D 249/18, G03F 7/16

(21) Anmeldenummer: **87810585.7**

(22) Anmeldetag: **12.10.87**

## (54) Lichtempfindliche Gemische.

(30) Priorität: **17.10.86 CH 4156/86**

(43) Veröffentlichungstag der Anmeldung:
**25.05.88 Patentblatt 88/21**

(45) Bekanntmachung des Hinweises auf die Patenterteilung:
**11.09.91 Patentblatt 91/37**

(84) Benannte Vertragsstaaten:
**CH DE FR GB IT LI NL**

(56) Entgegenhaltungen:
**EP-A- 0 031 296**
**FR-A- 2 115 374**
**US-A- 3 046 111**
**US-A- 3 531 414**
**US-A- 4 104 070**

**CHEMICAL ABSTRACTS, Band 93, Nr. 8, 25. August 1980, Seite 21, Zusammenfassung Nr. 72604t, Columbus, Ohio, US**

(73) Patentinhaber: **CIBA-GEIGY AG**
**Klybeckstrasse 141**
**CH-4002 Basel(CH)**

(72) Erfinder: **Bauer, Sigrid, Dr.**
**35, chemin de la Grangette**
**CH-1094 Paudex(CH)**

EP 0 268 553 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue o-Naphthochinondiazide, lichtempfindliche Gemische enthaltend diese Verbindungen, ein Verfahren zum Herstellen eines Positiv-Resistbildes sowie ein Verfahren zum Herstellen eines Negativ-Resistbildes durch Bild-Umkehr.

Aus der US-A 3,046,111 sind Umsetzungsprodukte aus Hydroxy-naphthoimidazolen mit Naphthochinondiazid-sulfonsäure(chlorid) als lichtempfindliche Komponente in positiv arbeitenden Photoresist-Materialien bekannt. Bei einem positiven Resistsystem wird das lichtempfindliche Material während der Belichtung derart abgewandelt, dass es im anschliessenden Entwicklungsschritt löslich wird. Die belichteten Stellen des Resistfilms werden beim Entwickeln entfernt, und die freien ungeschützten Flächen auf der Substratoberfläche entsprechen den lichtdurchlässigen Stellen auf der Photomaske. In der SU-A 732,294 sind Poly-(benzimidazolo-o-naphthochinondiazide) beschrieben, welche positive, aber keine negative Abbildungen ergeben.

Aus der US-A 4,104,070 ist ein Verfahren zum Herstellen eines Negativresistbildes unter Verwendung eines Positivresistmaterials enthaltend ein 1-Hyxdroxy-2-alkylimidazolin bekannt. Als negativ wird ein Photoresist bezeichnet, dessen belichtete Bereiche in einem geeigneten Entwickler unlöslich sind, während die unbelichteten Resistpartien von diesem weggelöst werden. Die "Bildumkehr" unter Verwendung eines Positivmaterials gemäss US-A 4,104,070 erfolgt dadurch, dass die Photoresistschicht nach der bildmässigen Belichtung eine ausreichende Zeit auf eine geeignete Temperatur erhitzt, anschliessend vollflächig belichtet und danach entwickelt wird.

Die vorliegende Erfindung betrifft neue lichtempfindliche o-Naphthochinondiazide der Formel (I) oder (II)

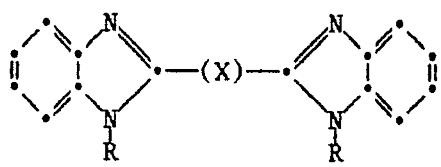

(I)

oder

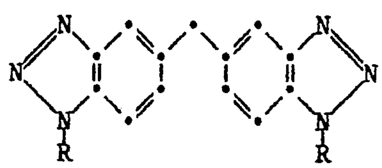

(II),

worin R Naphthochinon-(1,2)-diazid-(2)-4- oder -5-sulfonyl bedeutet und X eine geradkettige oder verzweigte, unsubstituierte oder mit einer oder zwei OH-Gruppen substituierte $C_1$-$C_{12}$ Alkylengruppe ist oder -CH = CH-bedeutet.

Beispiele für Gruppen X sind Methylen, Ethylen, Propylen-1,3; Propyliden-2,2; Butylen-1,4; Hexamethylen, Octamethylen, Dodecamethylen, 2-Hydroxypropylen-1,3 oder 2,4-Dihydroxyhexamethylen. Bevorzugt werden $C_1$-$C_8$ Alkylengruppen.

Die Herstellung der Verbindungen der Formel (I) oder (II) erfolgt auf an sich bekannte Weise, beispielsweise indem man die entsprechenden Verbindungen der Formel (I) oder (II), worin R Wasserstoff bedeutet (I* bzw. II*) mit etwa der stöchiometrischen Menge des Sulfonsäurechlorids von Naphthochinon-(1,2)-diazid, vorzugsweise in inerten organischen Lösungsmitteln in Gegenwart einer Base umsetzt. Die Sulfonsäuregruppe steht dabei in 4- oder 5-Stellung. Als Lösungsmittel eignet sich z.B. Dioxan, dem in manchen Fällen Dimethylformamid zugesetzt werden kann. Selbstverständlich sind für diese Umsetzung auch andere Lösungsmittel verwendbar. Die als säurebindende Mittel eingesetzten Basen sind beispielsweise Alkalihydroxide oder -carbonate, Pyridin oder Triethanolamin. Die Reaktion findet bei relativ tiefen Temperaturen, z.B. 20-40 °C glatt statt. Die Reinigung der Verbindungen der Formel (I) oder (II) kann, falls erwünscht auf bekannte Weise erfolgen, beispielsweise durch Umkristallisation oder Umfällen. Verfahren dieser Art sind z.B. in der US-A 3,046,111 oder in O. Süs et al., Angew. Chem. 74, 985 (1962) und den dort aufgeführten Literaturstellen beschrieben. Man kann die Verbindungen nach Ausfällen, Abfiltrieren und Trocknen jedoch auch tel quel einsetzen.

Die N-Heterocyclen der Formel (I), worin R Wasserstoff bedeutet, welche als Ausgangsstoffe dienen, sind bekannt. Deren Herstellung ist beispielsweise in Ann. 599, 44ff. (1956) beschrieben. Die Verbindung der Formel (II), worin R Wasserstoff ist, welche auch als Ausgangsstoff verwendet wird, ist ebenfalls bekannt und z.B. in der US-A 3,531,414 offenbart worden.

Die vorliegende Erfindung betrifft ferner die neuen Verbindungen der Formel (I) oder (II) enthaltende Gemische. Diese enthalten ein polymeres wasserunlösliches harzartiges Bindemittel, das sich in den für das erfindungsgemässe Gemisch verwendeten Lösungsmitteln löst und in wässrigen Alkalien bzw. Trialkylammoniumverbindungen löslich oder zumindest quellbar ist.

Die bei vielen Positiv-Kopiermaterialien auf Basis von Naphthochinondiaziden bewährten Novolak-Kondensationsharze haben sich als Zusatz auch bei den erfindungsgemässen Gemischen mit den neuen Naphthochinondiazidsulfonsäureamiden als besonders brauchbar und vorteilhaft erwiesen. Sie fördern die starke Differenzierung zwischen den belichteten und unbelichteten Bildelementen beim Entwickeln, besonders die höher kondensierten Harze mit substituierten Phenolen, z.B. Kresolen, als Formaldehyd-Kondensationspartner. Als weitere alkalilösliche bzw. in Alkali quellbare Bindemittel sind natürliche Harze, wie Schellack und Kolophonium, und synthetische Harze, wie Mischpolymerisate aus Styrol und Maleinsäureanhydrid oder Mischpolymerisate der Acrylsäure oder Methacrylsäure, insbesondere mit Acryl-oder Methacrylsäureestern, zu nennen.

Die Art und Menge des alkalilöslichen Harzes kann je nach Anwendungszweck verschieden sein; bevorzugt werden Anteile am Gesamtfeststoff zwischen 95 und 50, besonders bevorzugt 90-60 Gew.-%. Zusätzlich können noch zahlreiche andere Harze z.B. säuregruppenhaltige aber auch neutrale Zusatzharze mitverwendet werden. Geeignet sind Epoxide und Vinylpolymerisate wie Polyvinylacetate, Polyacrylate, Polyvinylacetate, Polyvinylether, Polyvinylpyrrolidone und die Mischpolymerisate der ihnen zugrundeliegenden Monomeren. Der günstigste Anteil an diesen Harzen richtet sich nach den anwendungstechnischen Erfordernissen und ihrem Einfluss auf die Entwicklungsbedingungen und beträgt im allgemeinen nicht mehr als 20 Gew.-% vom alkalilöslichen Harz. In geringen Mengen kann das lichtempfindliche Gemisch für spezielle Erfordernisse wie Flexibilität, Haftung, Glanz, Färbung und Farbumschlag etc. ausserdem noch Substanzen wie Polyglykole, Cellulosederivate wie Ethylcellulose, Netzmittel, Farbstoffe, Verlaufmittel, Füllstoffe, Haftvermittler, Weichmacher, und feinteilige Pigmente sowie bei Bedarf UV-Absorber enthalten. Besonders hervorzuheben ist auch der Zusatz von organischen Säuren bzw. Säurespendern, d.h. Verbindungen, welche unter dem Einfluss aktinischer Strahlung Säuren abspalten.

Zur Beschichtung eines geeigneten Schichtträgers werden die Gemische im allgemeinen in einem Lösungsmittel gelöst. Die Wahl der Lösungsmittel ist auf das vorgesehene Beschichtungsverfahren, die Schichtdicke und die Trocknungsbedingungen abzustimmen. Geeignete Lösungsmittel für das erfindungsgemässe Gemisch sind Ketone wie Methylethylketon, chlorierte Kohlenwasserstoffe wie Trichlorethylen und 1,1,1-Trichlorethan, Alkohole wie n-Propanol, Ether wie Tetrahydrofuran, Alkoholether wie Ethylenglykolmonoethylether und Ester wie Butylacetat. Es können auch Gemische verwendet werden, die zudem noch für spezielle Zweck Lösungsmittel wie Acetonitril, Dioxan, Cyclohexanon oder Dimethylformamid enthalten können. Prinzipiell sind alle Lösungsmittel verwendbar, die mit den Schichtkomponenten nicht irreversibel reagieren. Partialether von Glykolen, insbesondere Ethylenglykolmonoethylether, werden besonders bevorzugt.

Die lichtempfindlichen Gemische enthalten bevorzugt 2-35 Gew.-% und insbesondere 7-20 Gew.-% des Sensibilisators der Formel (I) oder (II) bezogen auf den Gesamtfeststoff.

Die erfindungsgemässen Zusammensetzungen eignen sich hervorragend als Beschichtungsmittel für Substrate aller Art, z.B. Holz, Textilien, Papier, Keramik, Glas, Kunststoffe, wie Polyester, Polyethylenterephthalat, Polyolefine oder Celluloseacetat, insbesondere in Form von Filmen, sowie von Metallen, wie Al, Cu, Ni, Fe, Zn, Mg oder Co, und von Si, $SiO_2$ oder Siliziumnitrid, bei denen durch bildmässiges Belichten eine Abbildung aufgebracht werden soll. Ein weiterer Gegenstand vorliegender Erfindung sind die beschichteten Substrate.

Die Erfindung betrifft ferner auch ein Verfahren zur Erzeugung von positiven Abbildungen, umfassend folgende Arbeitsschritte:

- Beschichtung eines Substrats mit einer strahlungsempfindlichen Zusammensetzung wie oben definiert,
- Belichtung des beschichteten Substrats mit einem vorbestimmten Muster aktinischer Strahlung, und
- Entwicklung des belichteten Substrats.

Die Belichtung der beschichteten Substrate kann z.B. erfolgen, indem man eine Lösung oder Suspension der Zusammensetzung mittels bekannten Beschichtungsverfahren auf ein Substrat gleichförmig aufbringt, z.B. durch Schleudern, Tauchen, Rakelbeschichtung, Vorhanggiessverfahren, Aufpinseln, Sprühen und Reverse Rollbeschichtung. Es ist auch möglich, die lichtempfindliche Schicht auf einen temporären,

flexiblen Träger zu bringen, und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Besonders vorteilhaft ist es, dass die erfindungsgemässen Zusammensetzungen in weit variablen Schichtdicken eingesetzt werden können.

Mögliche Einsatzgebiete der erfindungsgemässen Zusammensetzungen sind die Verwendung als Photoresists für die Elektronik (Galvanoresist, Aetzresist), die Herstellung von Druckplatten, wie Offsetdruckplatten, für den autotypischen Tiefdruck, den Rollodruck und ferner für die Herstellung von Siebdruckformen, der Einsatz beim Formteilätzen oder der Einsatz als Mikroresist bei der Herstellung integrierter Schaltkreise.

Dementsprechend unterschiedlich sind die möglichen Schichtträger und die Verarbeitungsbedingungen der beschichteten Substrate.

Für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Bei integrierten Schaltungen werden Si-Scheibchen als Trägermaterial verwendet.

Nach Beschichten wird das Lösungsmittel in der Regel durch Trocknen entfernt, und es resultiert eine Schicht des Photoresist auf dem Träger.

Nach der in üblicher Weise erfolgten bildmässigen Belichtung des Materials werden die belichteten Stellen des Photolackes durch Herauslösen in einem Entwickler entfernt.

Besonders bevorzugt als Entwickler werden wässrig alkalische Lösungen. Dazu zählen insbesondere wässrige Lösungen von Alkalimetallsilikaten, -phosphaten und -hydroxiden, sowie von Trialkylammoniumverbindungen. Diesen Lösungen können gegebenenfalls noch kleinere Mengen an Netzmitteln und/oder organischen Lösungsmitteln zugesetzt sein.

Typische organische Lösungsmittel, die den Entwicklerflüssigkeiten zugesetzt werden können, sind solche, die mit Wasser mischbar sind, wie beispielsweise 2-Ethoxyethanol oder Aceton, sowie Mischungen zweier oder mehrerer dieser Lösungsmittel.

Der Begriff 'Belichtung mit einem vorbestimmten Muster aktinischer Strahlung' beinhaltet sowohl die Belichtung durch eine Photomaske, die ein vorbestimmtes Muster enthält, beispielsweise ein Diapositiv, sowie die Belichtung durch einen Laserstrahl, der beispielsweise computergesteuert über die Oberfläche des beschichteten Substrates bewegt wird, und auf diese Weise ein Bild erzeugt.

Die Lichtempfindlichkeit der erfindungsgemässen Zusammensetzungen reicht in der Regel vom UV-Gebiet (ca. 250 nm) bis ca. 600 nm und umspannt somit einen sehr breiten Bereich. Als Lichtquellen kommen daher eine grosse Anzahl der verschiedensten Typen zur Anwendung. Es sind sowohl Punktlichtquellen als auch fläch enförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen und photographische Flutlichtlampen. Der Abstand zwischen Lampen und dem erfindungsgemässen Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser mit starken Emissionslinien (Ar-Laser) bei 457, 476, 488, 514, 528 nm. Bei dieser Art der Belichtung ist keine Photomaske im Kontakt mit der Photopolymerschicht mehr nötig; der gesteuerte Laser-Strahl schreibt direkt auf die Schicht. Hier ist die hohen Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die lichtempfindlichen Zusammensetzungen können gegebenenfalls auch Sensibilisatoren enthalten, um die spektrale Empfindlichkeit in einem bestimmten Bereich des elektromagnetischen Spektrums zu erhöhen.

Die Erfindung betrifft daher ausserdem die Verwendung der Zusammensetzungen, wie oben definiert, als Positiv-Photoresists für die Herstellung von positiv arbeitenden Kopierlacken, welche z.B. zur Herstellung von integrierten Schaltungen, Aetzreserven, Offsetdruckplatten, Farbprüffolien, Schablonen, Namensschilder etc. dienen, sowie von positiv arbeitenden Trockenresistfilmen.

Der erfindungsgemässe Einsatz der o-Naphtochinondiazide erbringt verglichen mit herkömmlichen Systemen insbesondere folgende Vorteile: Die erfindungsgemässen o-Naphtochinondiazide verleihen den mit ihnen hergestellten Schichten verbesserte Entwicklerresistenz und erhöhen die Lagerstabilität von Lösungen und Schichten. Es hat sich zudem gezeigt, dass Kupfer- oder oxidhaltige bzw. mit Hexamethyldisilazan behandelte Substrate Oberflächen mit den neuen Beschichtungslösungen besser benetzt werden

und riefenfreie Oberflächen ergeben. Die Entwickelbarkeit der Kopierschicht kann durch die Wahl der lichtempfindlichen Komponente auf eine beliebigen Entwickler eingestellt werden.

Eine Besonderheit stellt die Möglichkeit der Bildumkehr dar. Darunter wird verstanden, dass es möglich ist, mittels Anwendung des oben beschriebenen Positiv-Resistmaterials zu einem Negativ-Resistbild zu gelangen. Für den Anwender von Resistlösungen ist es einfacher, wenn Resists die Möglichkeit der Bildumkehr anbieten, da sonst die Resistlösungen erst durch geeignete Zusätze modifiziert werden müssen, was umständlich und unwirtschaftlich ist und ferner die Langzeitstabilität der Resistlösung verschlechtern kann.

Bei der Bildumkehr wird nach der bildmässigen Belichtung die Photoresistschicht eine ausreichende Zeit lang auf eine ausreichende Temperatur erhitzt und anschliessend die Schicht vollflächig belichtet und entwickelt. In einer vorteilhaften Ausgestaltung der Erfindung erfolgt die Erhitzung auf 100-130 °C, insbesondere auf 110-120 °C während 3 bis 20 Min., insbesondere während 5 bis 10 Min.

Die Entwicklung kann mittels der oben beschriebenen alkalischen Entwicklern durchgeführt werden. Mit diesem Verfahren erhält man ein Negativresistbild, das den mit üblichen Negativresistmaterialien erzeugten Resistbildern bezüglich Auflösung und Fehlstellen überlegen ist.

Die vorliegende Erfindung betrifft daher auch ein Verfahren zur Erzeugung negativer Abbildungen, umfassend folgende Arbeitsschritte:

- Beschichtung eines Substrats mit einem strahlungsempfindlichen Gemisch wie oben definiert,
- Belichten des beschichteten Substrats mit einem vorbestimmten Muster aktinischer Strahlung,
- Erhitzen des beschichteten Substrats,
- Vollflächiges Belichten des beschichteten Substrats mit aktinischer Strahlung und
- Entwicklung des belichteten Substrats.

Beispiel 1:

25 g (0,1 Mol) 5,5'-Methylen-bis-benztriazol werden in einer Mischung von 200 ml Dioxan und 100 ml Wasser gelöst. Dazu wird eine Lösung von 54 g (0,2 Mol) Naphthochinon-(1,2)-diazid(2)-5-sulfochlorid in 250 ml Dioxan gegeben. Dann werden 250 ml 10%ige Sodalösung unter heftigem Rühren tropfenweise hinzugefügt, wobei die Temperatur auf 32-37 °C ansteigt. (Dauer der Zugabe 2-3 Stunden). Zu der noch warmen Lösung werden anschliessend 2500 ml Wasser gegeben, wobei das Reaktionsprodukt ausfällt. Nach dem Erkalten wird die Verbindung abgenutscht, ausgiebig mit Wasser gewaschen und bei Raumtemperatur getrccknet.

Das gelbbraune Chinondiazid zersetzt sich langsam ab 125 °C.

Beispiel 2:

Analog der in Beispiel 1 beschriebenen Methode werden 274 g (1 Mol) 1,3-Bis-(2'-benzimidazolyl)-propan und 485 g (2 Mol) Naphthochinon-(1,2)-diazid-(2)-5-sulfonsäurechlorid in Dioxan umgesetzt. Das so erhaltene Produkt zersetzt sich ab 148 °C.

Beispiele 3-9:

Die folgenden Umsetzungsprodukte der Formel (I) wurden wie in Beispiel 1 beschrieben hergestellt, wobei pro Mol Benzimidazol-Verbindung jeweils 2 Mol Naphthochinon-(1,2)-diazid-(2)-5-sulfonsäurechlorid eingesetzt wurden:

| Beispiel | X in Formel (I) | zersetzt sich ab |
|---|---|---|
| 3 | $-(CH_2)_8-$ | 148°C |
| 4 | $-(CH_2)_4-$ | 149°C |
| 5 | $-(CH[OH])_2-$ | 205°C |
| 6 | $-CH=CH-$ (cis) | 135°C |
| 7 | $-CH=CH-$ (trans) | 146°C |
| 8 | $-CH_2-$ | 205°C |
| 9 | $-(CH_2)_6-$ | 141°C |

Die gemäss den Beispielen 1 bis 9 erhaltenen Umsetzungsprodukte können nach Ausfällen, Abnutschen und Trocknen ohne weitere Reinigung tel quel eingesetzt werden.

Beispiel 10: Es wird eine Beschichtungslösung aus

| | |
|---|---|
| 4,33 Gew.-t. | eines Poly(p-vinylphenols), Resin M® der Fa. Maruzen Oil, |
| 0,50 Gew.-t. | eines Epoxidharzes mit einem Epoxiwert von 2,0-2,2. |
| 1,00 Gew.-t. | Umsetzungsprodukt gemäss Beispiel 2, |
| 0,045 Gew.-t. | Kristallviolett (Colour Index No. 42 555) und |
| 15,60 Gew.-t. | eines Lösungsmittelgemisches aus Aethylglykol, Aethylglykolacetat und Methyläthylketon (im Verhältnis 2:2:1) |

zubereitet und mit einem Drahtrakel auf elektrochemisch aufgerauhtes Aluminium aufgetragen. (Trockenschichtdicke ca. 2µm).

Die lichtempfindliche Schicht wird unter einer photographischen Testvorlage, die u.a. einen 21-stufigen Graukeil der Firma Stouffer, einen UGRA-Offset-Testkeil 1982 und ein positives Rasterbild enthält, mit einer 5 KW Metallhalogenidlampe 6 Sekunden belichtet und mit dem Entwickler A, bestehend aus

| | |
|---|---|
| 40,0 Gew.-t. | $Na_3PO_4$, wasserfrei, |
| 20,0 Gew.-t. | Natrium-meta-silikat • 5 $H_2O$ und |
| 960,0 Gew.-t. | Wasser, deion., |

im Schaukelbad entwickelt.

Man erhält ein kontrastreiches Bild auf einem schleierfreien Hintergrund.

Im Falle einer gewünschten Bildumkehr wird wir folgt verfahren:

Die lichtempfindliche Schicht wird zunächst, wie schon oben beschrieben, 6 Sekunden belichtet, danach 5 Minuten bei 120 °C erwärmt, anschliessend 10 Sekunden vollflächig belichtet und abschliessend mit dem Entwickler A zu einem Komplementärbild der benutzten Vorlage entwickelt.

Beim Ersatz der o.a. lichtempfindlichen Verbindung durch das Umsetzungsprodukt aus 1 Mol 1,6-Bis-(2'-benzimidazolyl)-hexan und 2 Mol Naphthochinon-(1,2)-diazid-(2)-5-sulfochlorid (gemäss Beispiel 9) in der o.a. Beschichtungslösung werden die gleichen guten Ergebnisse erhalten. Es empfiehlt sich jedoch, den Entwickler:A 1:1 mit Wasser zu verdünnen.

Beispiel 11: Es wird eine Beschichtungslösung aus

| | |
|---|---|
| 10,50 Gew.-t. | eines Kresol-Formaldehyd-Novolaks mit einem Erweichungspunkt von 110-120 °C, |
| 1,25 Gew.-t. | Umsetzungsprodukt gemäss Beispiel 3, |
| 0,09 Gew.-t. | Kristallviolett (Colour Index No. 42 555) und |
| 35,60 Gew.-t. | eines Lösungsmittelgemisches aus Aethylglykol, Aethylenglykolacetat und Methyläthylketon (im Verhältnis 2:2:1). |

zubereitet und mit einem Drahtrakel auf mechanisch gebürstetes Aluminium aufgetragen. Danach wird wie in Beispiel 10 beschrieben belichtet und mit dem Entwickler A entwickelt. Das so erhaltene Bild ist einwandfrei.

Bildumkehr nach den in Beispiel 10 beschriebenen Verfahrensschritten ist auch hier möglich. Man erhält ein schleierfreies Komplementärbild der benutzten Vorlage.

Gleich gute Ergebnisse werden erhalten, wenn die o.a. lichtempfindliche Verbindung durch das Umsetzungsprodukt gemäss Beispiel 2 ersetzt wird.

Benutzt man als lichtempfindliche Verbindungen die Umsetzungsprodukte gemäss Beispiel 6 bzw. 9 und verfährt ansonsten wie in Beispiel 2 beschrieben, so erhält man qualitativ hochwertige Positiv- und Negativbilder der verwendeten Testvorlage. Zur Entwicklung ist der Entwickler A im Verhältnis 1:1 mit Wasser zu verdünnen.

Beispiel 12: Es wird eine Beschichtungslösung aus

| | |
|---|---|
| 6,40 Gew.-t. | eines Kresol-Formaldehyd-Novolaks mit einem Erweichungspunkt von 110-120 °C, |
| 0,20 Gew.-t | eines Polyvinylacetals mit ca. 70 % Vinylacetal-, 24-27 % Vinylalkohol und 1 % Vinylacetateinheiten, |
| 1,00 Gew.-t. | Naphthochinondiazid gemäss Beispiel 1, |
| 0,12 Gew.-t. | Naphthochinon-(1,2)-diazid-(2)-4-sulfonsäurechlorid |
| 0,06 Gew,-t. | Kristallviolett (Colour Index No. 42 555) und |
| 20,00 Gew.-t. | des in Beispiel 1 beschriebenen Lösungsmittelgemisches |

zubereitet und auf eine mit einer 35 µm starken Kupfer-Folie kaschierten Isolierplatte aufgebracht und getrocknet.

Zwecks Bebilderung wird die lichtempfindliche Schicht unter einer Strichvorlage 6 Sekunden mit einer 5 KW Metallhalogenidlampe belichtet und mit einem Entwickler, bestehend aus

| | |
|---|---|
| 5,3 Gew.-t. | Natrium-meta-silikat • 9 $H_2O$, |
| 3,4 Gew.-t. | Trinatriumphosphat • 12 $H_2O$, |
| 0,3 Gew.-t. | Natriumdihydrogenphosphat in |
| 91,0 Gew.-t. | Wasser, deion., |

entwickelt.

Geätzt wird mit einer handelsüblichen Fe-III-Chloridätze, gegen die die Bildbereiche resistent sind.

Beispiel 13: Es wird eine Beschichtungslösung aus

| | |
|---|---|
| 10,50 Gew.-t. | des in Beispiel 11 beschriebenen Novolaks, |
| 1,00 Gew.-t. | eines bromierten Epoxidharzes mit einem Epoxiwert von 2,0-2,2 und einem Bromgehalt von ca. 21,2 %, |
| 1,00 Gew.-t. | eines Styrol/Maleinsäureanhydrid-Copolymeren mit einem mittleren Molekulargewicht von 10 000 und einer Säurezahl von 190, |
| 1,25 Gew.-t. | Umsetzungsprodukt gemäss Beispiel 3, |
| 0,09 Gew.-t. | Kristallviolett und |
| 35,60 Gew.-t. | des in Beispiel 2 beschriebenen Lösungsmittelgemisches |

auf mechanisch gebürstetes Aluminium aufgetragen, getrocknet und dann unter einer photographischen Vorlage 6 Sekunden mit einer 5 KW Metallhalogenidlampe belichtet.

Entwickelt wird mit einer Lösung aus

| | |
|---|---|
| 50,0 Gew.-t. | Natrium-meta-silikat • 5 $H_2O$ und |
| 2,5 Gew.-t. | Natriumoxid in |
| 1000,0 Gew.-t. | Wasser, deion. |

Danach wird mit Wasser abgespült, durch Ueberwischen mit 1%-iger Phosphorsäure fixiert und mit einer Gummiarabikumlösung konserviert.

Die so hergestellte Platte eignet sich für den Offsetdruck und ist äusserst langlebig.

Bildumkehr entsprechend den Verarbeitungsschritten aus Beispiel 2 erbringt die ebenso guten Resultate.

Beispiel 14: Es werden 7 Beschichtungslösungen (I-VII) aus

| | |
|---|---|
| 21,00 Gew.-t. | des in Beispiel 11 beschriebenen Novolaks, |
| 2,00 Gew.-t. | des in Beispiel 10 beschriebenen Epoxidharzes, |
| 2,00 Gew.-t. | eines Methylmethacrylat/Methacrylsäure-Copolymeren mit einer Säurezahl 155 und einem mittleren Molekulargewicht von 160 000, |
| 2,50 Gew.-t. | eines Umsetzungsprodukts gemäss der Beispiele 2, 4, 6, 7, 8 und 9 |
| 0,18 Gew.-t. | Kristallviolett und |
| 71,20 Gew.-t. | des in Beispiel 1 beschriebenen Lösungsmittelgemisches |

zubereitet.

Zur Herstellung von Positiv- bzw. Negativ-bilder wird wie in Beispiel 2 beschrieben die Lösung in einer ersten Versuchsreihe auf gebürstetes Aluminium in einer zweiten Versuchsreihe auf Kupferkaschiertes Basismaterial, aufgetragen, getrocknet und belichtet. Als Entwickler eignet sich das Handelsprodukt Kodak micro positive developer® 809, mit Wasser im Verhältnis 1:1 verdünnt. Es werden auf diese Weise qualitativ einwandfreie Bilder erhalten.

Beispiel 15:

Zur Herstellung mikroelektronischer Schaltelemente wird wie folgt verfahren:
Es wird eine Beschichtungslösung aus

| | |
|---|---|
| 10,50 Gew.-t. | des in Beispiel 11 beschriebenen Novolaks, |
| 2,00 Gew.-t. | des in Beispiel 10 beschriebenen Epoxidharzes, |
| 0,60 Gew.-t. | 5,5'-Methylen-bis-benztriazol, |
| 1,25 Gew.-t. | Naphthochinondiazid gemäss Beispiel 1 und |
| 40,84 Gew.-t. | eines Lösungsmittelgemisches eines Aethylglykol und Aethylglykolacetat (1:1) |

zubereitet, durch ein 0,2 µm-Filter filtriert und durch Schleudern auf eine mit einer 0,2 µm dicken $SiO_2$-Schicht versehenen Siliciumscheibe dessen $SiO_2$-Schicht mit Hexamethyldisilazan als Haftvermittler beschichtet war, aufgebracht. Danach wird 10 Minuten bei 90° C im Umluftschrank getrocknet. Anschliessend wird abgekühlt und die Scheibe bei 23° C und 40-50 % relativer Luftfeuchtigkeit für die Belichtung konditioniert. Die Schichtdicke beträgt 1,2 µm. Belichtet wird während 6 Sekunden in einem Wafer-Kontaktbelichtungsgerät mit einer 200 Watt Hg-Hochdrucklampe. Als Testvorlage dient eine handelsübliche Chrommaske mit hochaufgelösten Linienmustern.

Entwickelt wird mit einem Entwickler der Fa. Shipley, Microposit© 303, der dazu mit Wasser im Verhältnis 1:4 verdünnt wurde.

Anschliessend wird mit Wasser abgespült und mit Stickstoff trocken geblasen.

Das Auflösungsvermögen beträgt 2 µm (für Linien mit entsprechender Breite und Abstand).

**Patentansprüche**

1. Verbindungen der allgemeinen Formel (I) oder (II)

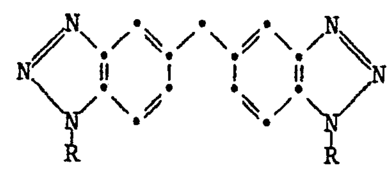

(II),

oder

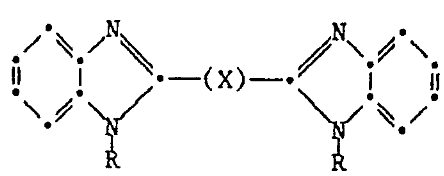

(I)

worin R Naphthochinon-(1,2)-diazid-(2)-4- oder -5-sulfonyl bedeutet und X eine geradkettige oder verzweigte, unsubstituierte oder mit einer oder zwei OH-Gruppen substituierte $C_1$-$C_{12}$ Alkylengruppe ist oder -CH=CH-bedeutet.

2. Verbindungen gemäss Anspruch 1, der Formel (I) worin X eine $C_1$-$C_8$ Alkylengruppe ist.

3. Strahlungsempfindliches Gemisch, enthaltend eine Verbindung der Formel (I) oder (II) gemäss Anspruch 1 und ein Bindemittel.

4. Strahlungsempfindliches Gemisch gemäss Anspruch 3, enthaltend als Bindemittel ein Phenolharz.

5. Strahlungsempfindliches Gemisch gemäss Anspruch 3, enthaltend zusätzlich ein Epoxidharz.

6. Strahlungsempfindliche Gemische gemäss Anspruch 3, enthaltend 2-35 Gew.-% einer Verbindung der Formel (I) oder (II).

7. Verfahren zur Erzeugung positiver Abbildungen, umfassend folgende Arbeitsschritte:
   - Beschichten eines Substrats mit einer strahlungsempfindlichen Zusammensetzung gemäss Anspruch 3,
   - Belichten des beschichteten Substrats mit einem vorbestimmten Muster aktinischer Strahlung, und
   - Entwicklung des belichteten Substrats.

8. Verfahren zur Erzeugung negativer Abbildungen, umfassend folgende Arbeitsschritte:
   - Beschichtung eines Substrats mit einem strahlungsempfindlichen Gemisch gemäß Anspruch 3,
   - Belichten des beschichteten Substrats mit einem vorbestimmten Muster aktinischer Strahlung,
   - Erhitzen des beschichteten Substrats,
   - Vollflächiges Belichten des beschichteten Substrats mit aktinischer Strahlung und
   - Entwicklung des belichteten Substrats.

9. Verfahren zur Herstellung von Verbindungen der Formel (I) oder (II) gemäss Anspruch 1, dadurch gekennzeichnet, dass man die entsprechende Verbindung der Formel (I) oder (II), worin R Wasserstoff ist (I*) bzw. (II*) mit etwa der stöchiometrischen Menge des Sulfonsäurechlorids von Naphthochinon-(1,2)-diazids umsetzt.

## Claims

1. A compound of the general formula (I) or (II)

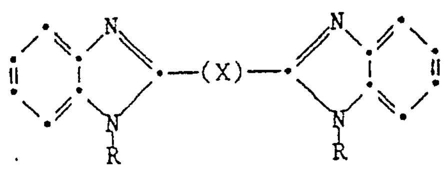

(I)

or

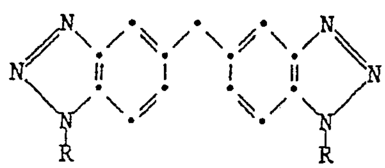

(II),

in which R is 1,2-naphthoquinone-2-diazide-4 or -5-sulfonyl and X is a straight-chain or branched $C_1$-$C_{12}$alkylene group which is unsubstituted or mono- or disubstituted by OH groups, or is -CH=CH-.

2. A compound according to claim 1, of the formula (I) wherein X is a $C_1$-$C_8$alkylene group.

3. A radiation-sensitive mixture, containing a compound of the formula (I) or (II) according to claim 1 and a binder.

4. A radiation-sensitive mixture according to claim 3, containing a phenolic resin as the binder.

5. A radiation-sensitive mixture according to claim 3, containing additionally an epoxide resin.

6. A radiation-sensitive mixture according to claim 3, containing 2-35% by weight of a compound of the formula (I) or (II).

7. A process for producing positive images, comprising the following working steps:
   - coating a substrate with a radiation-sensitive composition according to claim 3,
   - exposing the coated substrate to a predetermined pattern of actinic radiation and
   - developing the exposed substrate.

8. A process for producing negative images, comprising the following working steps:
   - coating a substrate with a radiation-sensitive mixture according to claim 3,
   - exposing the coated substrate to a predetermined pattern of actinic radiation,
   - heating the coated substrate,
   - exposing the full area of the coated substrate to actinic radiation and
   - developing the exposed substrate.

9. A process for preparing a compound of the formula (I) or (II) according to claim 1, which comprises reacting the corresponding compound of the formula (I) or (II) in which R is hydrogen, (I*) or (II*) respectively, with approximately the stoichiometric quantity of the sulfonic acid chloride of 1,2-naphthoquinone-diazide.

**Revendications**

1. Composés répondant aux formules générales. (I) ou (II) :

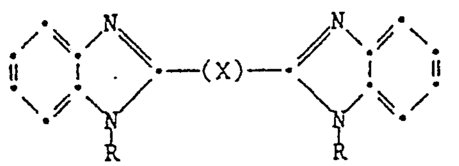

(I)

ou

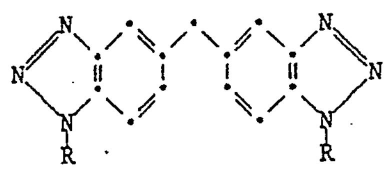

(II),

dans lesquelles R représente un groupe naphtoquinone-1,2 diazide -2 sulfonyl-4 ou -5, et X représente un groupe alkylène en $C_1$ à $C_{12}$, linéaire ou ramifié, non substitué ou portant un ou deux groupes OH comme substituants, ou bien X représente un groupe -CH=CH-.

2. Composés selon la revendication 1, répondant à la formule (I) dans laquelle X représente un groupe alkylène en $C_1$ à $C_8$.

3. Mélange photosensible, consistant en un composé de formule (I) ou (II) selon la revendication 1, et en un liant.

4. Mélange photosensible selon la revendication 3, contenant comme liant une résine phénolique.

5. Mélange photosensible selon la revendication 3, contenant en outre une résine époxyde.

6. Mélange photosensible selon la revendication 3, contenant 2 à 35 % en poids d'un composé de formule (i) ou (II).

7. Procédé pour produire des images positives, comportant les étapes opératoires suivantes :
   - revêtement d'un substrat à l'aide d'une composition photosensible selon la revendication 3,
   - exposition du substrat ainsi revêtu, avec un modèle prédéterminé, à l'action d'un rayonnement actinique, et

- développement du substrat exposé.

**8.** Procédé pour produire des images négatives, comprenant les étapes opératoires suivantes :
- revêtement d'un substrat par un mélange photosensible selon la revendication 3,
- exposition du substrat ainsi revêtu, avec un modèle prédéterminé, à l'action d'un rayonnement actinique,
- chauffage du substrat revêtu,
- exposition de la totalité de la surface du substrat revêtue à un rayonnement actinique, et
- développement du substrat exposé.

**9.** Procédé pour préparer des composés de formule (I) ou (II) selon la revendication 1, caractérisé en ce qu'on fait réagir le composé correspondant de formule (I) ou (II), dans laquelle R représente un atome d'hydrogène, (I*) ou (II*), avec la quantité approximativement stoechiométrique du chlorure d'acide naphtoquinone-1,2 diazide sulfonique.